# EUROPÄISCHE PATENTSCHRIFT

(11) **EP 0 844 991 B1**
(45) Veröffentlichungstag und Bekanntmachung des Hinweises auf die Patenterteilung: **22.12.1999**
(21) Anmeldenummer: 96929270.5
(22) Anmeldetag: 19.08.1996
(51) Int. Cl.: C07C 209/60

(54) **VERFAHREN ZUR HERSTELLUNG VON AMINEN AUS OLEFINEN AN BOR-BETA-ZEOLITHEN**
METHOD OF PREPARING AMINES FROM OLEFINS OVER BORON BETA-ZEOLITES
PROCEDE DE PREPARATION D'AMINES A PARTIR D'OLEFINES SUR DES ZEOLITES BETA AU BORE

(30) Priorität: 17.08.1995 DE 19530177
(43) Veröffentlichungstag der Anmeldung: 03.06.1998
(73) Patentinhaber: BASF AKTIENGESELLSCHAFT, 67056 Ludwigshafen (DE)
(72) Erfinder: DINGERDISSEN, Uwe, D-64342 Seeheim-Jugenheim (DE); KUMMER, Rudolf, D-67227 Frankenthal (DE); STOPS, Peter, D-67122 Altrip (DE); MÜLLER, Ulrich, D-67434 Neustadt (DE); HERRMANN, Jürgen, D-68307 Mannheim (DE); ELLER, Karsten, D-67059 Ludwigshafen (DE)
(86) Internationale Anmeldenummer: EP9603634
(87) Internationale Veröffentlichungsnummer: WO9707088

(56) Entgegenhaltungen:
- EP-A- 0 133 938
- EP-A- 0 721 934
- WO-A-92/20446
- US-A- 4 701 313

## Beschreibung

Die vorliegende Erfindung betrifft ein Verfahren zur Herstellung von Aminen durch Umsetzung von Ammoniak oder primären oder sekundären Aminen mit Olefinen bei erhöhten Temperaturen und Drükken in Gegenwart von Bor-BETA-Zeolithen.

Aus CA-A-2 092 964 ist ein Verfahren zur Herstellung von Aminen aus Olefinen bekannt, bei dem Aluminium-BETA-Zeolithe, die als kristalline Aluminosilikate bestimmter Zusammensetzung mit einer Porengröße von mehr als 5 Å definiert sind, eingesetzt werden.

Diese Katalysatoren lassen bezüglich Ausbeute oder Raum-Zeit-Ausbeute oder rasche Desaktivierung zu wünschen übrig.

Der vorliegenden Erfindung lag daher die Aufgabe zugrunde, den zuvor genannten Nachteilen abzuhelfen.

Demgemäß wurde ein neues und verbessertes Verfahren zur Herstellung von Aminen der allgemeinen Formel I 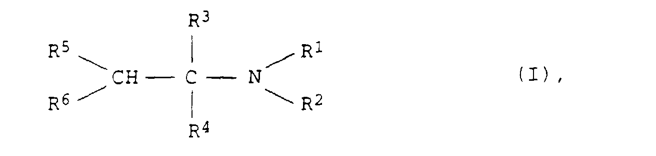 in der
- R¹, R², R³, R⁴, R⁵, R⁶: Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
- R¹ und R²: gemeinsam eine gesättigte oder ungesättigte C₃-bis C₉-Alkylendikette und
- R³ oder R⁵: C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 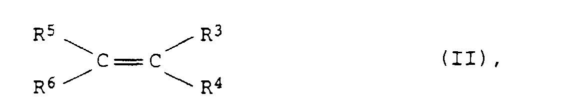 in der R³, R⁴, R⁵ und R⁶ die oben genannten Bedeutungen haben, mit Ammoniak oder primären oder sekundären Aminen der allgemeinen Formel III 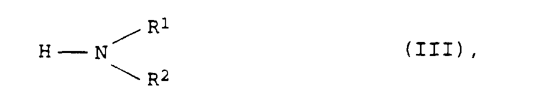 in der R¹ und R² die oben genannten Bedeutungen haben, bei Temperaturen von 200 bis 350°C und Drücken von 100 bis 300 bar in Gegenwart eines zeolithischen Katalysators gefunden, welches dadurch gekennzeichnet ist, daß man als zeolithischen Katalysator Bor-BETA-Zeolithe einsetzt.

Das erfindungsgemäße Verfahren kann wie folgt durchgeführt werden:

Das Olefin II und Ammoniak oder das primäre oder sekundären Amin III kann bei Temperaturen von 200 bis 350°C, bevorzugt 220 bis 330°C, besonders bevorzugt 230 bis 320°C und Drücken von 100 bis 300 bar, bevorzugt 120 bis 300 bar, besonders bevorzugt 140 bis 290 bar in Gegenwart eines Bor-BETA-Zeoliths als Katalysator z.B. in einem Druck-Reaktor umsetzt werden, und bevorzugt das erhaltene Amin abtrennt und die nichtumgesetzten Einsatzstoffe zurückführt werden.

Das vorliegende Verfahren zeichnet sich durch eine sehr gute Ausbeute bei hoher Selektivität und bei hoher Raum-Zeit-Ausbeute aus. Zudem ist die Desaktivierung der Katalysator zurückgedrängt worden.

Die Bor-BETA-Zeolithe zeichnen sich, speziell für diesen Typ der Reaktion (Direktaminierung von Olefinen), durch eine besonders günstige Anordnung der aciden Zentren in Verbindung mit der charakteristischen Größe des Mikroporensystems aus. Dies bewirkt eine hohe Aktivität und eine hohe Standzeit. Die vielfach beim Einsatz von Al-Zeolithen gefundenen Desaktivierungen der Katalysatoren werden der hohen Acidität der Al-Zeolithe zugeschrieben. Bor-Zeolithe haben bei gleicher Anzahl der aciden Zentren eine in der Stärke deutlich geringere Acidität. Durch die Verwendung von Bor-BETA-Zeolith lassen sich somit die Vorzüge des Beta-Zeolith-Porengefüges mit der erwünschten Acidität verknüpfen.

Das erfindungsgemäße Verfahren zeichnet sich dadurch aus, daß bereits mit niedrigem Ammoniak- bzw. Amin- Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine III zusammen mit dem Olefin II im molaren Verhältnis von 1:1 bis 5:1 gemischt einem Festbettreaktor zuführt und bei einem Druck von 100 bis 300 bar und einer Temperatur von 200 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt.

Man kann einfach oder mehrfach ungesättigte Olefine II, insbesondere solche mit 2 bis 10 C-Atomen bzw. deren Mischungen und Polyolefine als Ausgangsstoffe verwenden. Wegen der geringer ausgeprägten Polymerisationsneigung eignen sich Monoolefine, insbesondere Isobuten, Diisobuten, Cyclopenten, Cyclohexen, besser als Di- und Polyolefine, insbesondere Polyisobuten, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstigt das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins und des Katalysators abhängig und liegt meist im Bereich von 200 bis 350°C.

Als Katalysatoren für die Aminierung von Olefinen eignen sich Bor-BETA-Zeolithe, die z.B. nach Gaodeng Xuexiao Huaxue Xuebao (1993), 14(2), 159 bis 163 oder Gaodeng Xuexiao Huaxue Xuebao (1989), 10(7), 677 bis 682 oder WO-A-92/20446 hergestellt werden können.

Die Bor-BETA-Zeolithe, die bevorzugt in der H-Form eingesetzt werden, können als solche, oder aber auch mit einem Bindemittel im Verhältnis 98:2 bis 40:60 Gew.-%, zu Strängen oder Tabletten verformt werden. Als Bindemittel eignen sich verschiedene Aluminiumoxide, bevorzugt Boehmit, amorphe Aluminosilikate mit einem SiO₂/Al₂O₃-Verhältnis von 25:75 bis 95:5, Siliciumdioxid, bevorzugt hochdisperses SiO₂, Gemische aus hochdispersem SiO₂ und hochdispersem Al₂O₃, hochdisperses TiO₂ sowie Tone. Nach der Verformung werden die Extrudate oder Preßlinge zweckmäßig bei 110°C/16 h getrocknet und bei 200 bis 500°C/2 bis 16 h calciniert, wobei die Calcinierung auch direkt im Aminierungsreaktor erfolgen kann.

Zur Erhöhung der Selektivität, der Standzeit und der Anzahl der möglichen Regenerierungen kann man verschiedene Modifizierungen an zeolithischen Katalysatoren vornehmen.

Eine Modifizierung der Katalysatoren besteht darin, daß man die unverformten oder die verformten Zeolithe mit Alkalimetallen wie Na und K, Erdalkalimetallen wie Ca, Mg, Erdmetallen wie Tl, Übergangsmetallen wie z.B. Mn, Fe, Mo, Cu, Zn, Cr, Edelmetallen und/oder seltenen Erdmetallen wie z.B. La, Ce oder Y ionenaustauschen bzw. dotieren kann.

Eine vorteilhafte Ausführungsform besteht darin, daß man die verformten Bor-BETA-Zeolithe in einem Strömungsrohr vorlegt und bei 20 bis 100°C, z.B. ein Halogenid, Acetat, Oxalat, Citrat oder Nitrat der oben beschriebenen Metalle in gelöster Form darüberleitet. Ein derartiger Ionenaustausch kann z.B. an der Wasserstoff-, Ammonium- und Alkaliform der Zeolithe vorgenommen werden.

Eine andere Möglichkeit der Metallaufbringung auf die Zeolithe besteht darin, daß man das zeolithische Material, z.B. mit einem Halogenid, Acetat, Oxalat, Citrat, Nitrat oder Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten Zeolithen kann eine Nachbehandlung mit Wasserstoff und/oder mit Wasserdampf günstig sein.

Eine weitere Möglichkeit der Modifizierung besteht darin, daß man das zeolithische Material - verformt oder unverformt - einer Behandlung mit Säuren, wie Salzsäure (HCl), Flußsäure (HF), Oxalsäure (H₂C₂O₄), Schwefelsäure (H₂SO₄), Phosphorsäure (H₃PO₄) oder deren Gemischen unterwirft.

Eine besondere Ausführungsform besteht darin, daß man das zeolithische Pulver vor seiner Verformung mit einer der genannten Säuren 0,001 n bis 2 n, bevorzugt 0,05 bis 0,5 n 1 bis 100 Stunden unter Rückfluß behandelt. Nach Abfiltrieren und Auswaschen wird in der Regel bei 100 bis 160°C getrocknet und bei 200 bis 600°C calciniert. Eine weitere besondere Ausführungsform liegt in einer Säure-Behandlung der Zeolithe nach ihrer Verformung mit Bindemittel. Hierbei wird der Zeolith in der Regel 1 bis 3 Stunden zwischen 60 und 80°C mit einer 3 bis 25%igen, insbesondere mit einer 12 bis 20%igen Säure behandelt, anschließend ausgewaschen, bei 100 bis 160°C getrocknet und bei 200 bis 600°C calciniert.

Eine andere Möglichkeit der Modifizierung ist gegeben durch einen Austausch mit Ammoniumsalzen, z.B. mit NH₄Cl oder mit Mono-, Di- oder Polyaminen. Hierbei wird der mit Bindemittel verformte Zeolith in der Regel bei 60 bis 80°C mit 10 bis 25%iger, bevorzugt 20%iger NH₄Cl-Lösung 2 h kontinuierlich in gewichtsmäßiger Zeolith/Ammonchlorid-Lösung von 1:15 ausgetauscht und danach bei 100 bis 120°C getrocknet.

Eine weitere Modifikation, die an den erfindungsgemäßen Bor-Beta-Zeolithen vorgenommen werden kann, ist eine Deborierung, bei der ein Teil der Boratome durch Silicium ersetzt wird oder die Zeolithe durch beispielsweise hydrothermale Behandlung in ihrem Borgehalt abgereichert werden. An eine hydrothermale Deborierung schließt sich vorteilhafterweise eine Extraktion mit Säuren oder Komplexbildnern an, um gebildetes Nichtgitterbor zu entfernen. Der Ersatz von Bor durch Silicium kann beispielsweise mit Hilfe von (NH₄)₂SiF₆ oder SiCl₄ erfolgen.

Aus der US-A-4 701 313 ist der Austausch von Bor durch Silicium in Bor-BETA-Zeolithen mit SiCl₄ bekannt.

Die Katalysatoren kann man als Stränge mit Durchmesser von z.B. 1 bis 4 mm oder als Tabletten mit z.B. 3 bis 5 mm Durchmesser für die Aminierung der Olefine einsetzen.

Aus dem beispielsweise zu Strängen verformten Katalysator kann man durch Mahlen und Sieben ein Wirbelgut in der Größe von 0,1 bis 0,8 mm erhalten.

Die Substituenten R¹, R², R³, R⁴, R⁵ und R⁶ in den Verbindungen I, II und III haben folgende Bedeutungen:
R¹, R², R³, R⁴, R⁵, R⁶
   - Wasserstoff,
   - C₁- bis C₂₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
   - C₂- bis C₂₀-Alkenyl, bevorzugt C₂- bis C₁₂-Alkenyl, besonders bevorzugt C₂- bis C₈-Alkenyl wie Vinyl und Allyl,
   - C₂- bis C₂₀-Alkinyl, bevorzugt C₂- bis C₁₂-Alkinyl, besonders bevorzugt C₂- bis C₈-Alkinyl wie C₂H und Propargyl,
   - C₃- bis C₂₀-Cycloalkyl, bevorzugt C₃- bis C₁₂-Cycloalkyl, besonders bevorzugt C₅- bis C₈-Cycloalkyl wie Cyclopentyl, Cyclohexyl, Cycloheptyl und Cyclooctyl,
   - C₄- bis C₂₀-Alkyl-cycloalkyl, bevorzugt C₄- bis C₁₂-Alkylcycloalkyl, besonders bevorzugt C₅- bis C₁₀-Alkyl-cycloalkyl wie 2-Methylcyclopentyl und 4-Methylcyclohexyl,
   - C₄- bis C₂₀-Cycloalkyl-alkyl, bevorzugt C₄- bis C₁₂-Cycloalkyl-alkyl, besonders bevorzugt C₅- bis C₁₀-Cycloalkylalkyl wie Cyclopentyl-methyl und Cyclohexyl-methyl,
   - Aryl wie Phenyl, 1-Naphthyl und 2-Naphthyl, bevorzugt Phenyl,
   - C₇- bis C₂₀-Alkylaryl, bevorzugt C₇- bis C₁₆-Alkylaryl, bevorzugt C₇- bis C₁₂-Alkylphenyl wie 2-Methylphenyl, 3-Methylphenyl, 4-Methylphenyl, 2-Ethylphenyl, 3-Ethylphenyl und 4-Ethylphenyl,
   - C₇- bis C₂₀-Aralkyl, bevorzugt C₇- bis C₁₆-Aralkyl, bevorzugt C₇- bis C₁₂-Phenalkyl wie Phenylmethyl, 1-Phenylethyl und 2-Phenylethyl,
R¹ und R²
   - gemeinsam eine gesättigte oder ungesättigte C₃- bis C₉-Alkylendikette, bevorzugt -(CH₂)₄-, -(CH₂)₅-, -(CH₂)₇- und -CH=CH-CH=CH-,
R³ oder R⁵
   - C₂₁- bis C₂₀₀-Alkyl, bevorzugt C₄₀- bis C₂₀₀-Alkyl wie Polybutyl, Polyisobutyl, Polypropyl, Polyisopropyl und Polyethyl, besonders bevorzugt Polybutyl und Polyisobutyl,
   - C₂₁- bis C₂₀₀-Alkenyl, bevorzugt C₄₀- bis C₂₀₀-Alkenyl, besonders bevorzugt C70- bis C₁₇₀-Alkenyl,
R³ und R⁵
   - gemeinsam eine C₂- bis C₁₂-Alkylendikette, bevorzugt eine C₃-bis C₈-Alkylendikette, besonders bevorzugt -(CH₂)₃-, -(CH₂)₄-, -(CH₂)₅-, -(CH₂)₆- und -(CH₂)₇-, insbesondere -(CH₂)₃- und -(CH₂)₄-.

### Beispiele

### Katalysatorsynthese

60 g Borsäure (H₃BO₃) wurden in einer Lösung aus 206 g dest. Wasser und 344 g 40 %iger Tetraethylammoniumhydroxid-Lösung gelöst und in einen Autoklaven überführt. Nach Zugabe von 550 g Ludox® AS 40 (42 % SiO₂, Du Pont) wurde dieser verschlossen und 216 h bei 150°C kristallisiert. Der gebildete Bor-Beta-Zeolith wurde abfiltriert, mit dest. Wasser neutral gewaschen, 24 h bei 120°C getrocknet und 5 h bei 500°C calciniert.

### Beispiele Katalysatorverstrangung

### Katalysator A

100g Bor-Beta-Zeolith (SiO₂/B₂O₃ = 20) wurden mit 23,8 g Ludox® (42 % SiO₂. Du Pont) und 5 g Stärke versetzt. Im Kneter wurde die Mischung kompaktiert und unter vorsichtigem Wasserzusatz (64 ml) verknetet. Die Knetzeit betrug 60 min. In einer Strangpresse wurden mit einem Preßdruck von 90 bar 2mm Stränge erzeugt, bei 110°C 60 h getrocknet und anschließend 16 h bei 500°C kalziniert.

### Katalysator B

60g Bor-Beta-Zeolith (SiO₂/B₂O₃ = 20) wurden mit 40g Boehmit und 2g Ameisensäure versetzt. Im Kneter wurde die Mischung kompaktiert und unter vorsichtigem Wasserzusatz (57 ml) verknetet. Die Knetzeit betrug 60 min. In einer Strangpresse wurden mit einem Preßdruck von 100 bar 2mm Stränge erzeugt, bei 110°C 16 h getrocknet und anschließend 16 h bei 500°C kalziniert.

### Katalysator C

Katalysator C wurde analog Katalysator A hergestellt, jedoch mit einem Bor-Beta-Zeolithen mit einem SiO₂/B₂O₃-Verhältnis von 19.

### Katalysator D

Katalysator D wurde analog Katalysator B hergestellt, jedoch mit einem Bor-Beta-Zeolithen mit einem SiO₂/B₂O₃-Verhältnis von 19.

### Katalysator E

Katalysator E wurde analog Katalysator D hergestellt, jedoch mit nur 20 g Boehmit.

### Katalysator F

Katalysator F wurde analog Katalysator B hergestellt, jedoch mit einem Bor-Beta-Zeolithen mit einem SiO₂/B₂O₃-Verhältnis von 19.

### Katalysator G

Katalysator G wurde analog Katalysator A hergestellt, jedoch mit einem Bor-Beta-Zeolithen mit einem SiO₂/B₂O₃-Verhältnis von 23.

### Katalysator H

Katalysator H wurde analog Katalysator B hergestellt, jedoch mit einem Bcr-Beta-Zeolithen mit einem SiO₂/B₂O₃-Verhältnis von 23.

### Aminierungsbeispiele

In einen 0,3 1-Rührautoklaven wurden jeweils 10 g der oben beschriebenen Katalysatoren gegeben und nach dem Verschließen die Olefine und Ammoniak bzw. Amine aufgedrückt. Die Menge an Einsatzprodukt wurde entweder so bemessen, daß bei der gewählten Reaktionstemperatur der gewünschte Druck als Eigendruck der Reaktionspartner erreicht wurde oder es wurde noch zusätzlich Stickstoff aufgepreßt. Das molare Verhältnis von Ammoniak/Amin zu Olefin wurde von 1:1 bis 5:1 variiert, die Reaktionsdauer auf 16 h festgelegt.

Der Austrag wurde getrennt nach Flüssig- und Gasphase gaschromatographisch untersucht. Die in den Tabellen aufgeführten Umsätze beziehen sich immer auf das Olefin; die angegebenen Selektivitäten beziehen sich auf die Hauptprodukte: Cyclohexylamin aus Cyclohexen, Cyclopentylamin aus Cyclopenten, tert.-Butylamin aus Isobuten.

Wahlweise wurden die Versuche in einem Rohrreaktor (6 mm Innendurchmesser) unter isothermen Bedingungen bei 260 bis 300°C und einem Druck von 280 bar mit einem Gemisch aus Isobuten und Ammoniak im molaren Verhältnis von 1:1,5 durchgeführt. Die Reaktionsprodukte wurden im Gaschromatographen analysiert.

Die Ergebnisse mit den verschiedenen Katalysatoren sind in den Tabellen 1 bis 3 zusammengestellt.

**Tabelle 1:**

| tert.-Butylamin (NH₃ : C₄H₈ = 1,5) | | | | | | | | | |
|---|---|---|---|---|---|---|---|---|---|
| Katalysator | Anteil Verstrangungshilfsmittel | | Druck | Temperatur | tert.-Butyl-Ausbeute [Gew.-%] | | | | Litergewicht |
| | Al₂O₃ [%] | SiO₂ [%] | [bar] | [°C] | WHSV 0,5 g/g·h | WHSV 0,7 g/g·h | WHSV 1,5 g/g·h | WHSV 3 g/g·h | [kg/l] |
| A | | 9 | 280 | 270 | | 22,67 | 21,44 | 18,33 | 0,50 |
| B | 40 | | 280 | 260 | | 22,95 | | | 0,61 |
| B | 40 | | 280 | 270 | | 21,66 | 20,52 | 17,71 | 0,61 |
| B | 40 | | 280 | 280 | | | 18,12 | 16,83 | 0,61 |
| B | 40 | | 280 | 300 | | | | 12,80 | 0,61 |
| A | | 9 | 280 | 280 | | 18,83 | 18,78 | 17,98 | 0,50 |
| A | | 9 | 280 | 300 | | | | 12,28 | 0,50 |
| A | | 9 | 280 | 260 | 25,86 | 24,04 | 20,35 | 16,34 | 0,50 |
| C | | 9 | 280 | 270 | | 21,63 | 21,27 | 20,87 | 0,45 |
| D | 40 | | 280 | 270 | | 22,80 | 20,34 | 18,21 | 0,52 |
| E | 20 | | 280 | 270 | | 22,00 | 20,95 | 18,20 | 0,43 |
| F | 40 | | 280 | 270 | | 22,24 | 21,08 | 18,78 | 0,54 |
| G | | 9 | 280 | 270 | | 21,30 | 20,45 | 18,55 | 0,48 |
| H | 40 | | 280 | 270 | | 20,93 | 20,51 | 18,44 | 0,57 |

**Tabelle 2:**

| Cyclopentylamin (NH₃: C₅H₈ = 3) | | | |
|---|---|---|---|
| Katalysator | Druck | Temperatur | Cyclohexylamin |
| | [bar] | [°C] | [Mol-%] |
| B-Beta, SiO₂/B₂O₃=20 | 425 | 300 | 7,6 |

**Tabelle 3:**

| Cyclohexylamin (NH₃: C₆H₁₀ = 3) | | | |
|---|---|---|---|
| Katalysator | Druck | Temperatur | Cyclohexylamin |
| | [bar] | [°C] | [Mol-%] |
| B-Beta, SiO₂/B₂O₃=20 | 380 | 300 | 7,1 |

Der Katalysator F wurde darüber hinaus noch in einem kontinuierlichen Versuch auf Standzeit untersucht. 50 g Katalysator F wurden in einen Rohrreaktor (15 mm Innendurchmesser) mit eingebauter Thermohülse zur Messung der Innentemperatur eingebaut, wobei oberhalb und unterhalb der Katalysatorschüttung (72 cm Höhe) jeweils Steatit als Inertmaterial eingefüllt wurde. Die Heizung des Reaktors und die Vorheizung des Eduktgemisches erfolgte jeweils über Ölbäder, der Reaktorzulauf erfolgte von oben und das Produktgemisch wurde zweistufig entspannt (280 bar → 20 bar → 1 bar). Bei 275°C und 280 bar, einer WHSV von 3 g/g·h, einem NH₃-zu-Isobutenverhältnis von 1,5:1 wurde über einen Zeitraum von 1100 h eine konstante TBA-Ausbeute von 17,3 Gew.-% erhalten, ohne daß eine beginnende Desaktivierung zu beobachten gewesen wäre.

## Patentansprüche

1. Verfahren zur Herstellung von Aminen der allgemeinen Formel I 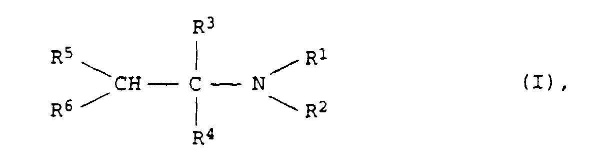 in der
R¹, R², R³, R⁴, R⁵, R⁶ Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
R¹ und R² gemeinsam eine gesättigte oder ungesättigte C₃- bis C₉-Alkylendikette und
R³ oder R⁵ C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 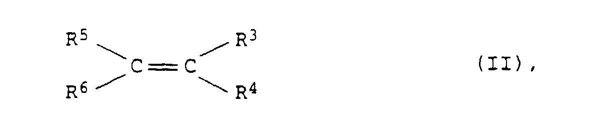 in der R³, R⁴, R⁵ und R⁶ die oben genannten Bedeutungen haben, mit Ammoniak oder primären oder sekundären Aminen der allgemeinen Formel III 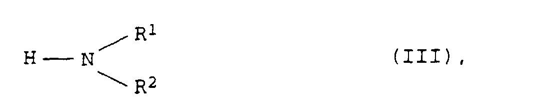 in der R¹ und R² die oben genannten Bedeutungen haben, bei Temperaturen von 200 bis 350°C und Drücken von 100 bis 300 bar in Gegenwart eines zeolithischen Katalysators, dadurch gekennzeichnet, daß man als zeolithischen Katalysator Bor-BETA-Zeolithe einsetzt.

2. Verfahren zur Herstellung von Aminen I nach Anspruch 1, dadurch gekennzeichnet, daß man Bor-BETA-Zeolithe in der H-Form einsetzt.

3. Verfahren zur Herstellung von Aminen I nach Anspruch 1 bis 2, dadurch gekennzeichnet, daß man das gebildete Amin I abtrennt und die nichtumgesetzten Einsatzstoffe II und III zurückführt.

4. Verfahren zur Herstellung von Aminen nach den Ansprüchen 1 bis 3, dadurch gekennzeichnet, daß man als Olefin II Isobuten, Diisobuten, Cyclopenten, Cyclohexen oder Polyisobuten einsetzt.

5. Verfahren zur Herstellung von Aminen nach den Ansprüchen 1 bis 4, dadurch gekennzeichnet, daß man als zeolithischen Katalysatoren Bor-BETA-Zeolithe, die mit einer Säure, insbesondere aus der Gruppe Salzsäure, Flußsäure, Schwefelsäure, Oxalsäure und Phosphorsäure, behandelt sind, einsetzt.

6. Verfahren zur Herstellung von Aminen nach den Ansprüchen 1 bis 5, dadurch gekennzeichnet, daß man als zeolithische Katalysatoren Bor-BETA-Zeolithe, die mit einem oder mehreren Übergangsmetallen dotiert sind, einsetzt.

7. Verfahren zur Herstellung von Aminen nach den Ansprüchen 1 bis 6, dadurch gekennzeichnet, daß man als zeolithische Katalysatoren Bor-BETA-Zeolithe, die mit einem oder mehreren Elementen der Seltenen Erden dotiert sind, einsetzt.

8. Verfahren zur Herstellung von Aminen nach den Ansprüchen 1 bis 7, dadurch gekennzeichnet, daß man als zeolithische Katalysatoren Bor-BETA-Zeolithe, die mit einem oder mehreren Elementen aus der Gruppe der Alkali-, Erdalkali- oder Erdmetalle dotiert sind, einsetzt.

9. Verfahren zur Herstellung von Aminen nach den Ansprüchen 1 bis 8, dadurch gekennzeichnet, daß man als zeolithische Katalysatoren Bor-BETA-Zeolithe in der Ammoniumform einsetzt.

10. Verfahren zur Herstellung von Aminen nach den Ansprüchen 1 bis 9, dadurch gekennzeichnet, daß man als zeolithische Katalysatoren Bor-BETA-Zeolithe einsetzt, die mit einem Bindemittel verformt und bei Temperaturen von 200 bis 600°C calciniert sind.

11. Verfahren zur Herstellung von Aminen nach den Ansprüchen 1 bis 10, dadurch gekennzeichnet, daß man als zeolithische Katalysatoren Bor-Beta-Zeolithe einsetzt, die nach ihrer Synthese in ihrem Borgehalt abgereichert wurden.

## Claims

1. A process for preparing amines of the general formula I 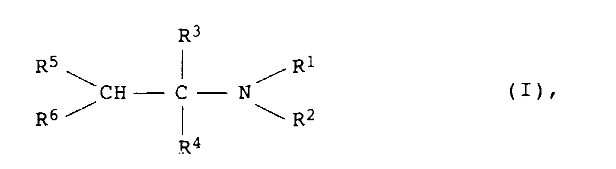 where
R¹, R², R³, R⁴, R⁵, R⁶ are hydrogen, C₁-C₂₀-alkyl, C₂-C₂₀-alkenyl, C₂-C₂₀-alkynyl, C₃-C₂₀-cycloalkyl, C₄-C₂₀-alkylcycloalkyl, C₄-C₂₀-cycloalkylalkyl, aryl, C₇-C₂₀-alkylaryl or C₇-C₂₀-aralkyl,
R¹ and R² are together a saturated or unsaturated, divalent C₃-C₉-alkylene chain and
R³ and R⁵ are each C₂₁-C₂₀₀-alkyl, C₂₁-C₂₀₀-alkenyl or together are a divalent C₂-C₁₂-alkylene chain,
by reacting olefins of the general formula II 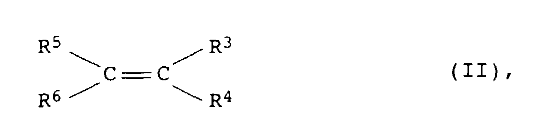 where R³, R⁴, R⁵ and R⁶ are as defined above, with ammonia or primary or secondary amines of the general formula III 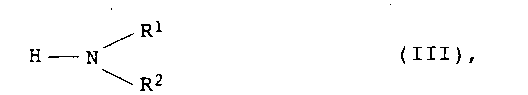 where R¹ and R² are as defined above, at from 200 to 350°C and pressures of from 100 to 300 bar in the presence of a zeolitic catalyst, wherein the zeolitic catalyst used is a boron BETA-zeolite.

2. A process for preparing amines I as claimed in claim 1, wherein the boron BETA-zeolite is used in the H form.

3. A process for preparing amines I as claimed in claim 1 or 2, wherein the amine I formed is separated off and the unreacted starting materials II and III are recirculated.

4. A process for preparing amines as claimed in any of claims 1 to 3, wherein the olefin II used is isobutene, diisobutene, cyclopentene, cyclohexene or polyisobutene.

5. A process for preparing amines as claimed in any of claims 1 tc 4, wherein the zeolitic catalyst used is a boron BETA-zeolite which has been treated with an acid, in particular an acid selected from the group consisting of hydrochloric acid, hydrofluoric acid, sulfuric acid, oxalic acid and phosphoric acid.

6. A process for preparing amines as claimed in any of claims 1 to 5, wherein the zeolitic catalyst used is a boron BETA-zeolite which has been doped with one or more transition metals.

7. A process for preparing amines as claimed in any of claims 1 to 6, wherein the zeolitic catalyst used is a boron BETA-zeolite which has been doped with one or more elements of the rare earths.

8. A process for preparing amines as claimed in any of claims 1 to 7, wherein the zeolitic catalyst used is a boron BETA-zeolite which has been doped with one or more elements selected from the group consisting of the alkali metals, alkaline earth metals or earth metals.

9. A process for preparing amines as claimed in any of claims 1 to 8, wherein the zeolitic catalyst used is a boron BETA-zeolite in the ammonium form.

10. A process for preparing amines as claimed in any of claims 1 to 9, wherein the zeolitic catalyst used is a boron BETA-zeolite which has been shaped using a binder and calcined at from 200 to 600°C.

11. A process for preparing amines as claimed in any of claims 1 to 10, wherein the zeolitic catalyst used is a boron BETA-zeolite which has had its boron content reduced after synthesis.

## Revendications

1. Procédé de préparation d'amines de formule générale I 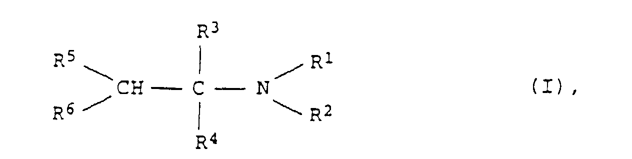 dans laquelle
R¹, R², R³, R⁴, R⁵, R⁶ représentent chacun l'hydrogène, un groupe alkyle en C1-C20, alcényle en C2-C20, alcynyle en C2-C20, cycloalkyle en C3-C20, alkylcycloalkyle en C4-C20, cycloalkylalkyle en C4-C20, aryle, alkylaryle en C7-C20 ou aralkyle en C7-C20,
R¹ et R² représentent ensemble une chaîne divalente alkylène saturée ou insaturée en C3-C9 et
R³ et R⁵ représentent chacun un groupe alkyle en C21-C200, alcényle en C21-C200 ou, ensemble, une chaîne divalente alkylène en C2-C12
par réaction d'oléfines de formule générale II 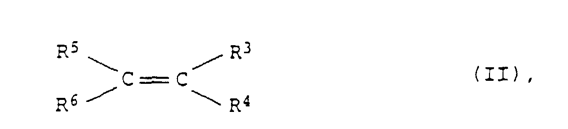 dans laquelle R³, R⁴, R⁵ et R⁶ ont les significations indiquées ci-dessus, avec l'ammoniac ou des amines primaires ou secondaires de formule générale III 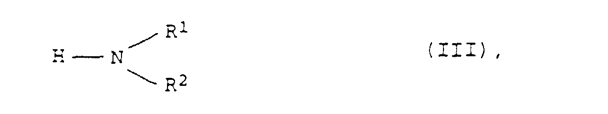 dans laquelle R¹ et R² ont les significations indiquées ci-dessus, à des températures de 200 à 350°C sous des pressions de 100 à 300 bar, en présence d'un catalyseur zéolitique, caractérisé par le fait que l'on utilise en tant que catalyseur zéolitique une zéolite bêta de bore.

2. Procédé pour la préparation des amines I selon la revendication 1, caractérisé par le fait que l'on utilise la zéolite bêta de bore sous la forme H.

3. Procédé pour la préparation des amines I selon la revendication 1 à 2, caractérisé par le fait que l'on sépare l'amine I formée et on recycle les composants de départ II et III non convertis.

4. Procédé pour la préparation d'amines selon les revendications 1 à 3, caractérisé par le fait que l'oléfine II mise en oeuvre est l'isobutène, le diisobutène, le cyclopentène, le cyclohexène ou un polyisobutène.

5. Procédé de préparation d'amines selon les revendications 1 à 4, caractérisé par le fait que l'on utilise en tant que catalyseurs zéolitiques des zéolites bêta de bore traitées par un acide, en particulier un acide du groupe de l'acide chlorhydrique, de l'acide fluorhydrique, de l'acide sulfurique, de l'acide oxalique et de l'acide phosphorique.

6. Procédé pour la préparation d'amines selon les revendications 1 à 5, caractérisé par le fait que l'on utilise en tant que catalyseurs zéolitiques des zéolites bêta de bore dopées par un ou plusieurs métaux de transition.

7. Procédé pour la préparation d'amines selon les revendications 1 à 6, caractérisé par le fait que l'on utilise en tant que catalyseurs zéolitiques des zéolites bêta de bore dopées par un ou plusieurs éléments des terres rares.

8. Procédé pour la préparation d'amines selon les revendications 1 à 7, caractérisé par le fait que l'on utilise en tant que catalyseurs zéolitiques des zéolites bêta de bore, dopées par un ou plusieurs éléments du groupe des métaux alcalins, des métaux alcalino-terreux ou des métaux terreux.

9. Procédé pour la préparation d'amines selon les revendications 1 à 8, caractérisé par le fait que l'on utilise en tant que catalyseurs zéolitiques des zéolites bêta de bore sous la forme ammonium.

10. Procédé pour la préparation d'amines selon les revendications 1 à 9, caractérisé par le fait que l'on utilise en tant que catalyseurs zéolitiques des zéolites bêta de bore façonnées avec un liant et calcinées à des températures de 200 à 600°C.

11. Procédé pour la préparation d'amines selon les revendications 1 à 10, caractérisé par le fait que l'on utilise en tant que catalyseurs zéolitiques des zéolites bêta de bore qui, après leur synthèse, ont été appauvries en bore.
